# EUROPEAN PATENT APPLICATION

(11) **EP 3 895 602 A1**
(43) Date of publication of application: **20.10.2021**
(21) Application number: 20169451.0
(22) Date of filing: 14.04.2020
(51) Int. Cl.: A61B 5/00, A61B 5/0205

(54) **PATIENT DATA ANALYSIS METHOD AND SYSTEM**

(71) Applicant: Biosensors Beyond Borders Ltd, London WC1B 3RE (GB)
(72) Inventor: Bozoky, Zoltan Egon, London W2 4RL (GB); Lafferty-Smith, Erin, Beckenham, BR3 4HJ (GB); Schmit, Claude, 5425 Gostingen (LU)
(74) Representative: Dehns

(57) **Abstract**

A monitoring device 100 is provided for measuring patient data from a patient. The monitoring device 100 comprises measurement analysis software 200 for analysing the patient data, and is configured to: a. send patient data to a software provider; and b. receive one or more updates to the measurement analysis software from the software provider 301. A software provider 301 for providing the measurement analysis software for the monitoring device is also provided. The software provider 301 is configured to: a. receive patient data from the monitoring device 100; and b. send one or more updates to the measurement analysis software 200 to the monitoring device 100, wherein the one or more updates to the measurement analysis software 200 are based on an analysis of the received patient data.

## Description

The present invention relates to the field of patient (e.g. clinically useful) data analysis. A method and a system for analysing patient data (e.g. online and offline) are presented, which may perform detection and/or assessment of health.

In settings where disease levels are well above what is seen in other populations, assessment of and/or monitoring of changes to patient status is an essential component of illness management. Despite this, a lack of screening tools adapted for use in resource-limited settings, combined with delayed presentation, means that patients who are ill, or are becoming ill, may be missed or not treated sufficiently rapidly despite possible indications of clinical deterioration.

According to a first aspect, there is provided a monitoring device for measuring patient (e.g. clinically useful) data from a patient, the monitoring device comprising measurement analysis software for analysing the measured patient data, and the monitoring device being configured to: (a) send measured patient data to a software provider; and (b) receive one or more updates to the measurement analysis software from the software provider.

Thus, a monitoring device is provided which can itself both measure patient data from a patient and then analyse that measured data using measurement analysis software comprised by the device. As such, a single device can both take measurements and analyse those measurements, meaning that a user or operator can use the device and obtain a measurement analysis with relatively little or basic training.

In addition the monitoring device can (e.g. when suitably connected via a wired or wireless network/data connection) send (e.g. some or all of) the measured patient data to a software provider and receive one or more updates to the measurement analysis software from the software provider. As such, the device can share (e.g. some or all of) its measurements with a software provider (e.g. for use in software optimisation as described below) and also receive software updates from the software provider, such that the device can use up-to-date software algorithms.

The monitoring device preferably comprises one or more sensors for measuring the patient data.

The measurement analysis software is preferably configured to display a result of analysing the patient data with the measurement analysis software on the monitoring device, e.g. on a screen, such that it can be easily and quickly viewed by an operator.

The measurement analysis software is preferably also configured to provide an interpretation of a result of analysing the patient data with the measurement analysis software, and the analysis software is preferably further configured to display the interpretation on the monitoring device.

The interpretation may comprise an indication of a health status of the patient, for example. The indication (e.g. measurement, assessment and/or detection) of a health status of the patient may comprises an indication of one or more disease(s), e.g. the possibility, a likelihood, and/or a status of the patient having one or more disease(s). The indication of one or more diseases may include an indication of potentially life-threatening infectious and/or cardiometabolic factors and/or conditions (e.g. glucose, cholesterol levels, malnutrition).

The interpretation may comprise an indication of a spectral classification. The indication of a spectral classification may be used, for example, for measuring or determining the (or some of the) unique spectral features representative of (a) disease.

The interpretation may contain information and/or data which may comprise one or more numbers (e.g. percentages or numbers on a (pre-defined) scale such as from 0-1) indicating the (likely) heath status of the patient. As such, the monitoring device may provide an interpretation of the measured data, on which the operator and/or patient may then act (e.g. sending the patient for further testing or treatment, or scheduling further monitoring).

The measurement analysis software, or further software provided on the monitoring device, preferably comprises software for encrypting and/or anonymising measured patient data. As such, (only) encrypted and/or anonymised data may be sent to the software provider, thereby ensuring data security and/or patient confidentiality.

The patient data preferably comprises spectral or optical data (e.g. data obtained from one or more spectroscopy or optical measurements) and the measurement analysis software is preferably configured to analyse the spectral or optical data to obtain skin property/properties data. This property/properties data may include a skin phototype or pigmentation indication; collagen; melanin; and/or skin hydration data. This may be used in the analysis and/or interpretation of other spectral or light-based (optical) measurements made by the monitoring device, e.g. for calibration to a patient's particular skin colour or pigmentation, e.g. at a particular measurement site on a patient's body.

According to a further aspect, there is provided a software provider for providing measurement analysis software for a monitoring device (e.g. as described above) for measuring patient data from a patient, the measurement analysis software being software for analysing the measured patient data, and the software provider being configured to: (a) receive measured patient data from the monitoring device; and (b) send one or more updates to the measurement analysis software to the monitoring device, wherein the one or more updates to the measurement analysis software are based (at least partly) on an analysis of the received measured patient data. The patient data on which a software update is based may have been obtained with the same monitoring device to which the software update is sent, one or more different (further/additional) monitoring devices, or a combination of both the same monitoring device to which the software update is sent and one or more different (further/additional) monitoring devices.

As such, a software provider is preferably provided to receive measured patient data from a monitoring device (or a plurality of such monitoring devices) and provide one or more updates to measurement analysis software used by the monitoring device(s), based (at least partly) on received measured patient data.

The software provider is preferably provided on a remote device, e.g. remote to the monitoring device(s).

The software provider may comprise a quarantine database for storing received measured patient data from the monitoring device. By storing the received measured patient data in a quarantine database, this can allow for the data to be checked or reviewed, e.g. checked that it has a corresponding structure to other received data, before it is stored in a further database of the software provider, e.g. from which it can be analysed.

The software provider preferably comprises software for analysing the measured patient data received from the monitoring device. The software for analysing the measured patient data received from the monitoring device preferably comprises one or more models and/or protocols (e.g. medical-mathematical models, look-up tables, statistical learning models and/or neural network(s)). Thus, the measurement analysis software used by the monitoring device may be trained, e.g. using machine learning algorithms, on (previously) measured data. This can help to optimise the measurement analysis software.

The software provider preferably further comprises a database of data from one or more datasets and/or one or more data processing and analysis pipelines (e.g. data measured by one or more devices other than the monitoring device described above). Such data could have been obtained from clinical trials, for example. Such data may be used to determine disease insights.

The software for analysing the measured patient data received from the monitoring device is also preferably configured to analyse the data from one or more datasets and/or data processing and analysis pipelines and to provide the one or more updates to the measurement analysis software based on the analysis of the measured patient data received from the monitoring device and the analysis of the data from one or more datasets and/or data processing and analysis pipelines. By including data from one or more datasets and/or data processing and analysis pipelines, this can help to optimise the measurement analysis software.

The one or more updates to the measurement analysis software may comprise one or more updates to model parameters used by the measurement analysis software and/or one or more updates to protocols used by the measurement analysis software, for example. The protocols may be interpretation protocols, for example, which may specify how a measurement result is to be interpreted.

According to a further aspect, there is provided a health monitoring system, the system comprising: (a) one or more monitoring devices for measuring patient data from a patient, each monitoring device comprising measurement analysis software for analysing the patient data measured by the monitoring device; and (b) a software provider; wherein the health monitoring system is configured such that the one or more monitoring devices can send patient data measured by the one or more monitoring devices to the software provider, and the software provider can provide one or more updates to the measurement analysis software to the one or more monitoring devices.

As such, a health monitoring system may be provided comprising both one or more monitoring devices for measuring patient data from a patient and a software provider for providing one or more updates to measurement analysis software used by the one or more monitoring devices.

Preferably, the one or more monitoring devices are as described above or below, e.g. with any of the optional or preferred features.

Preferably, the software provider is as described above or below, e.g. with any of the optional or preferred features.

According to a further aspect, there is provided a method of monitoring patient health, the method comprising: (a) measuring patient data from a patient with a monitoring device; (b) analysing the measured patient data with measurement analysis software; (c) sending measured patient data to a software provider; and (d) receiving one or more updates to the measurement analysis software from the software provider.

The method may be performed with the monitoring device as described herein, e.g. with any of the optional or preferred features.

The method preferably further comprises displaying a result of analysing the patient data with the measurement analysis software on the monitoring device, e.g. on a screen or display.

The method preferably further comprises providing an interpretation of a result of analysing the patient data with the measurement analysis software. The interpretation may comprise an indication of a health status of the patient such as an indication of (e.g. the patient having or the status of) one or more disease(s), including potentially life-threatening infectious and/or cardiometabolic factors and/or conditions. The interpretation may comprise an indication of a spectral classification such as a spectral classification that may be used for measuring the or some (e.g. unique) spectral features representative of disease(s).

The method preferably further comprises encrypting and/or anonymising measured patient data.

The patient data preferably comprises one or more of mechanical, electrical, biochemical, molecular and/or skin property(ies) data.

The patient data (e.g. and in particular the skin property(ies) data) may comprise spectral and/or waveform data. The method preferably comprises analysing the spectral and/or waveform data. The method preferably further comprises analysing the spectral and/or waveform data to obtain skin property/properties data.

According to a further aspect, there is provided a method of providing a software update to measurement analysis software for analysing patient data measured by a monitoring device, the method comprising: (a) receiving measured patient data from the/a monitoring device; and (b) sending one or more updates to the measurement analysis software to the/a monitoring device, wherein the one or more updates to the measurement analysis software are based (at least partly) on an analysis of the received measured patient data. The received measured patient data may be from the same monitoring device to which the software update is sent or a different monitoring device.

The method may comprise quarantining the received measured patient data.

The analysis of the received measured patient data preferably comprises using one or more methods which may comprise using medical-mathematical models, look-up tables, statistical learning models and/or neural network(s).

The one or more updates to the measurement analysis software are preferably also based on an analysis of data from one or more datasets and/or data processing and analysis pipelines (e.g. as measured by one or more devices other than the monitoring device as described herein), such as data obtained from clinical trials, for example.

The one or more updates to the measurement analysis software may comprise one or more updates to model parameters used by the measurement analysis software and/or one or more updates to protocols used by the measurement analysis software.

According to a further aspect, there is provided a computer program product comprising computer readable instructions that, when run on a computer (or one or more processors), is configured to cause one or more processors to perform any of the methods (or parts of the methods) described herein.

Thus, for greater speed and efficiency, the methods or aspects of the methods are preferably performed on, or implemented by, a computer or one or more processors.

The methods in accordance with the present invention may be implemented at least partially using software e.g. computer programs. It will thus be seen that when viewed from further aspects, the present invention provides computer software specifically adapted to carry out the methods herein described when installed on data processing means (e.g. one or more processors), a computer program element comprising computer software code portions for performing the methods herein described when the program element is run on data processing means, and a computer program comprising code means adapted to perform all the steps of a method or of the methods herein described when the program is run on a data processing system. The data processor may be a microprocessor system, a programmable FPGA (field programmable gate array), etc.

The invention also extends to a computer software carrier comprising such software which when used to operate a processor or microprocessor system comprising data processing means causes in conjunction with said data processing means said processor or system to carry out the steps of the methods of the present invention. Such a computer software carrier could be a physical storage medium such as a ROM chip, RAM, flash memory, CD ROM or disk, or could be a signal such as an electronic signal over wires, an optical signal or a radio signal such as to a satellite or the like.

It will further be appreciated that in some embodiments, not all steps of the methods of the invention need be carried out by computer software and thus from a further broad aspect the present invention provides computer software and such software installed on a computer software carrier for carrying out at least one of the steps of the methods set out herein.

The present invention may accordingly suitably be embodied as a computer program product for use with (or within) a computer system. Such an implementation may comprise a series of computer readable instructions fixed on a tangible medium, such as a non-transitory computer readable medium, for example, diskette, CD ROM, ROM, RAM, flash memory or hard disk. It could also comprise a series of computer readable instructions transmittable to a computer system, via a modem or other interface device, either over a tangible medium, including but not limited to optical or analogue communications lines, or intangibly using wireless techniques, including but not limited to microwave, infrared or other transmission techniques. The series of computer readable instructions embodies all or part of the functionality previously described herein.

Those skilled in the art will appreciate that such computer readable instructions can be written in a number of programming languages for use with many computer architectures or operating systems. Further, such instructions may be stored using any memory technology, present or future, including but not limited to, semiconductor, magnetic, or optical, or transmitted using any communications technology, present or future, including but not limited to optical, infrared, or microwave. It is contemplated that such a computer program product may be distributed as a removable medium with accompanying printed or electronic documentation, for example, shrink wrapped software, pre-loaded with a computer system, for example, on a system ROM or fixed disk, or distributed from a server or electronic bulletin board over a network, for example, the Internet or World Wide Web.

Preferred embodiments of the invention will now be described by way of example only and with reference to the accompanying drawings, in which:
Fig. 1 is a schematic diagram illustrating the high-level relationship between a user, a monitoring device, measurement and analysis software and a remote device;
Fig. 2 is a schematic diagram illustrating a monitoring device with a software system that can be used in conjunction with a monitoring device;
Fig. 3 is a schematic diagram illustrating measurement analysis software;
Fig. 4 is a schematic or flow diagram illustrating an operational workflow of measurement and analysis software;
Figs. 5 is a schematic or flow diagram illustrating an analysis flow using measurement and analysis software;
Fig. 6 is a schematic diagram illustrating a software provider;
Fig. 7 is a schematic diagram illustrating some examples of various users of a machine learning system;
Fig. 8 is a schematic diagram illustrating a patient data analysis system and access gateways therein;
Fig. 9 is a schematic diagram illustrating a data repository;
Fig. 10 is a schematic diagram illustrating a convolutional neural network;
Fig. 11 is a schematic flowchart illustrating authorised user use of a machine learning system; and
Fig. 12 is a schematic flowchart illustrating automated measurement and analysis software use of a machine learning system.

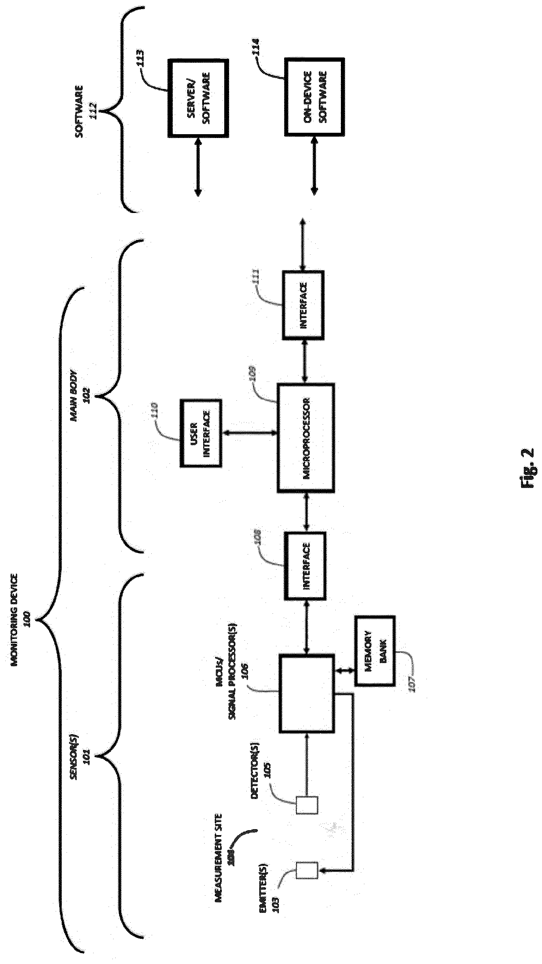

A monitoring device 100 is illustrated in Fig. 2. The monitoring device 100 is a portable, medical, mechanical, electrical and/or optical device. It is designed to be used primarily on a patient's lower arm (below the elbow), hands and/or finger(s).

In some embodiments, the device 100 includes an accessory that may be attached, stand-alone and/or connected to the rest of the monitoring device 100 for additional functionality. This accessory is arranged and configured to make measurements on a patient's hand.

The monitoring device 100 can measure, monitor, detect and/or assess a number of different clinically useful (patient) data.

The monitoring device 100 comprises sensor(s) 101 and a main body 102.

The sensor(s) 101 are provided and arranged on the main body 102 in such a way as to accommodate and take measurements from a patient's lower arm (below the elbow), hands and/or finger(s). The main body 102 is arranged and configured to support and take measurements from a patient's lower arm (below the elbow), hands and/or finger(s).

The sensor(s) 101 may comprise one or more emitter(s) 103 which each act as a source of mechanical, electrical and/or optical detection transmitted to a measurement site 104 on a patient. The sensor(s) 101 may also comprise one or more detector(s) 105, which capture and measure mechanical, electrical, biochemical and/or molecular outputs from the measurement site 104.

Measurements made by the emitter(s) 103 and detector(s) 105 may be in combination (at the same time) or sequential, or a combination of both (e.g. some measurements may be made at the same time and some may be sequential).

The sensor(s) 101 of the monitoring device 100 also (each) include MCUs/processor(s) 106 and memory bank(s) 107.

The MCUs/processor(s) 106 can control the emitter(s) 103 and/or detector(s) 105 and adapt (e.g. pre-process as described above) and process (e.g. as described above) the signal received so that it can be analysed by the monitoring device 100 and on-device software 202 (which is described below).

As also shown in Fig. 2, memory bank(s) 107 are provided in the sensor(s) 101, which serve as a data storage location(s) for the MCUs/processor(s) 106, among other uses.

The memory bank(s) 107 and MCUs/processor(s) 106 contained in the monitoring device 100 are for controlling the measurement/monitoring process, storing the measured data, and analysing clinically useful measurements taken by the monitoring device 100.

In some sensor(s) 101, the detector(s) 105 and MCUs/processor(s) 106 may collect and adapt (e.g. pre-process) skin property/properties signal(s) or data received by one or more of the detector(s) 105, such that it can be adjusted according to a range of tissue types (e.g. different skin pigmentations).

The monitoring device 100 also comprises in its main body 102 communication means (or interface 108 and/or 111) for communicating, e.g. wirelessly and/or via a wired connection, with on-device measurement analysis software and/or a remote server/software. Communicating with on-device measurement analysis software and/or a remote server/software may comprise sending and/or receiving data.

The main body 102 comprises a user interface 110 contained within the monitoring device 100. However, in alternative embodiments the monitoring device 100 can be provided without a user interface 110 and can provide an output to another display or system. The main body 102 can include various other components such as a microprocessor 109 or other components not illustrated in Fig 2.

The user interface 110 allows an operator to control the monitoring device 100, as described in more detail below. In some embodiments, the user interface 110 comprises buttons, a touch screen display, a liquid crystal display (LCD), or the like for controlling the monitoring device 100. The user interface 110 may allow a status (e.g. battery level, monitoring status) of the monitoring device 100 and control options to be displayed to a user, e.g. for selection via the user interface 110 or to perform other operations (e.g. control data communications).

An interface 108 is provided between the sensor(s) 101 and the components of the main body 102, e.g. microprocessor 109. To communicate and/or share the patient (e.g. mechanical, electrical, biochemical and/or molecular) data between the sensor(s) 101 and the main body 102, the interface 108 can be a wired connection e.g. serial bus port and/or universal serial bus port (USB).

The user interface 110 communicates with the sensor(s) 101 via the microprocessor 109 and interface 108. The user interface 110 can also communicate with further, e.g. remote, devices via the microprocessor 109 and interface 111.

The one or more sensor(s) 101 on the monitoring device 100 may comprise mechanical, electrical and/or optical sensor(s) arranged to measure patient data such as mechanical, electrical, biochemical and/or molecular data and/or skin property data.

One or more multi-wavelength optical sensor(s) are provided in the monitoring device 100, each comprising an emitter(s) 103 and/or detector(s) 105 and are arranged to perform transmission, absorption and/or reflectance spectroscopy in the UV, visible, near-infrared, mid-infrared and far-infrared ranges, (i.e. wavelengths of 10 nm - 1 mm). Patient data can be measured (or determined from measurements made) from the wavelength dependent measurement of light. Any or all of the following patient data may be determined: oxygen saturation; haemoglobin (Hb) concentration; Hb derivatives (oxygenated Hb, deoxygenated Hb, carboxyHb, metHb); haematocrit; blood clotting; blood velocity; blood viscosity; blood flow rate; relative blood flow; oxygen consumption rate; platelet adhesion and/or platelet rigidity; water and/or other molecules and when quantified and/or combined may be used to measure a range of factors and/or conditions and/or transcutaneous bilirubin.

The device 100 may comprise a thermometer (e.g. as one of its sensors) for measuring the temperature at one or more measurement sites 104. Such temperature measurements may be used to determine a temperature gradient, perfusion and/or circulation across a patent's lower arm (below the elbow), hands and/or finger(s). This is physiological data. The thermometer may be a digital thermometer.

The one or more optical sensor(s) may be arranged to measure (or measure data which can be used to determine) one or more of the following skin, property/properties data including: skin phototype; collagen; and/or skin hydration. These are skin/tissue data.

The position of the sensors 101 (especially the emitter(s) 103 and/or detector(s) 105) is changeable so that the device 100 can be adjusted to accommodate different lower arm (below the elbow), hands and/or finger(s) lengths and can allow some/all/any light-based measurements made by the monitoring device 100 to be adjusted to account for different or particular skin property/properties data.

Skin humidity also be measured at the lower arm (below the elbow), hands and/or finger(s) with a moisture or humidity sensor provided in the monitoring device 100.

The monitoring device 100 may also comprise a cardioelectrical and/or cardiomechanical sensor(s) and one or more body composition sensor(s).

One or more cardioelectrical and/or cardiomechanical sensor(s) and body composition sensor(s) can be in the small accessory for the hand.

The one or more cardioelectrical and/or cardiomechanical sensor(s) are arranged to measure the flow of electricity when cardiac cells produce a wave of depolarisation that can be sensed across the entire vascular tree. The sensor(s) are arranged to derive patient data related to electrical impulses from the heart and may detect abnormalities and/or complications. These are cardioelectrical and/or cardiomechanical data.

By including one or more sensor(s) for measuring cardioelectrical and/or cardiomechanical data in the monitoring device, along with sensors for measuring other kinds of patient data (e.g. as described above), cardioelectrical and/or cardiomechanical data may then be used in an analysis of and involving the other kinds of patient data, allowing such an analysis to be improved compared, for example, with situations where cardioelectrical and/or cardiomechanical data are not available.

The one or more body composition sensor(s) comprise an alternating electrical current of very low intensity, sent from metal electrodes through the body to detect and measure the ability of biological tissues to impede the electrical current. The body composition sensor(s) are arranged to measure lean mass and fluid volumes (e.g. total body water, intracellular fluid and/or extracellular fluid). The body composition sensor(s) may also be used to measure abnormal loss in lean body mass, the unbalanced shift in body fluids (e.g. severe dehydration and/or water retention), breathing and/or patient data related to the timing and magnitude of the arterial wave.

These cardioelectrical and/or cardiomechanical sensor(s) and body composition sensor(s) can be provided in a region on the device 100 where, in use, a patient's measurement site 104 would be located, so that it can perform the above measurements on the patient's lower arm (below the elbow), hands and/or finger(s).

The monitoring device 100 is arranged to support and take measurements from the patient's lower arm (below the elbow), hands and/or finger(s).

The monitoring device 100 may comprise one or more thermometer(s), described above, to measure the patient's temperature at at least two different locations. The thermometer(s) may be digital thermometer(s).

The monitoring device 100 further may comprise one or more optical sensor(s), which is/are arranged to measure a variety of biological molecules and/or disease-specific variations in molecular states on a patient's lower arm (below the elbow), hands and/or finger(s). The optical sensor(s) may measure the unique spectral features representative of disease in blood, interstitial fluid and/or tissue. This could be used to assess or detect life-threatening infections and/or cardiometabolic factors and/or conditions. These may be biochemical and/or molecular data.

One or more of the emitter(s) 103 and/or detector(s) 05 may be outside the device casing(s) and attached by wires and/or tubes at a fixed length and may be designed to be stored in the one or more device casing(s).

The monitoring device 100 may comprise cardioelectrical and/or cardiomechanical, body composition and/or mechanical sensor(s) and/or emitter(s) and detector(s) provided in the form of one or more moveable parts such that it/they may be moveable with respect to the rest of the monitoring device 100.

One or more mechanical sensor(s) could be an electromechanical sensor based on a strain gauge load cell for obtaining strength measurement(s) electronically. In particular, such a sensor could be used to measure continuous muscle function and maximum grip strength. These measurements may be used to derive a patient's muscle function and muscle strength data.

One or more mechanical sensor(s) may be provided for measuring patient data related to the timing and magnitude of the arterial wave. Such sensors could comprise a cuff in the form of an oscillometric and/or inflatable cuff. An inflation means (e.g. a pump and/or bulb) for the cuff can allows for manual inflation of the cuff. The cuff is arranged to make measurements of a patient's lower arm (below the elbow), hands and/or finger(s). In particular, it is arranged to measure the travelling wave caused by cardiac contraction and reflection back from the periphery, which is influenced by the mechanical properties of the arteries, directly reflecting the status of the aorta, that may change early in the course of disease. These are vascular, cardiovascular, cardiac and/or aortic data.

The cuff and inflation means may be outside the device casing in the form of a moveable part and can be attached or otherwise connected to a patient appropriately and used for measuring, detecting and/or assessing patient measurements.

Thus, the monitoring device 100 (or similar devices) can allow various mechanical, electrical, biochemical, molecular and/or skin property(ies) data to be measured and stored in memory in the device 100.

The data measured by the device 100 (i.e. by various sensors in the device 100) includes any or all of the following:
- The travelling wave caused by cardiac contraction and reflection back from the periphery
- Oxygen saturation
- Haemoglobin (Hb) derivatives (Oxygenated Hb, Deoxygenated Hb, CarboxyHb, MetHb)
- Hb concentration
- Haematocrit
- Blood clotting
- Blood velocity
- Blood viscosity
- Blood flow rate
- Relative blood flow
- Oxygen consumption rate
- Platelet adhesion
- Platelet rigidity
- Transcutaneous bilirubin
- Perfusion and/or circulation
- The flow of electricity when cardiac cells produce a wave of depolarisation
- Patient data related to electrical impulses from the heart
- Lean mass
- Total body water
- Intracellular fluid
- Extracellular fluid
- Phase angle
- Breathing
- Patient data related to the timing and magnitude of the arterial wave
- Continuous muscle function
- Maximum grip strength
- Temperature gradient
- Biological molecules and/or disease-specific variations in molecular states
- The unique spectral features representative of disease
- Skin property/properties data including:
   ∘ Collagen
   ∘ Melanin
   ∘ Skin hydration

The travelling wave caused by cardiac contraction and reflection back from the periphery can be used to derive patient data related to the timing and magnitude of the arterial wave and can directly reflect the status of the aorta, that may change early in the course of disease.

The flow of electricity when cardiac cells produce a wave of depolarisation can be sensed across the entire vascular tree and can be used to derive patient data related to electrical impulses from the heart and may detect abnormalities and/or complications.

Strength measurements can be used to derive continuous muscle function and/or muscle strength data.

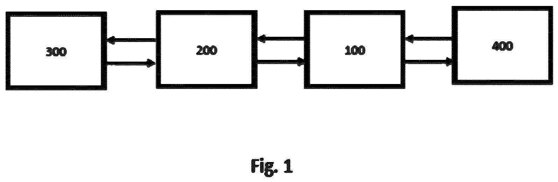

Fig. 1 is a schematic diagram illustrating the high-level relationship between a user 400, a monitoring device 100, measurement and analysis software 200, and a remote device 300.

The measurement and analysis software 200 is provided in one or more processors located in the monitoring device 100 to analyse and interpret the patient data measured by the monitoring device 100, and to provide an analysis and interpretation result, which can be displayed on the user interface 110 of the monitoring device 100. The monitoring device 100 (or its measurement and analysis software 200) can also communicate, when appropriately connected, e.g. via a mobile data, Wi-Fi or wired data connection, to a remote device 300, for example, to send measured patient data from the device 100 to the remote device 300 and/or to receive analysis software updates at the device 100 from the remote device 300.

The measurement and analysis software 200 provided on the monitoring device 100 can perform analysis and interpretation of the patient data measured by the device 100. This results in an analysis and interpretation result which is displayed to the user 400 on the user interface 110.

A software system 112 is provided for analysing data measured with the monitoring device 100. As illustrated schematically in Fig. 2 the software system 112 includes at least one server 113 with server software. The server 113 can store data (including data measured by the monitoring device 100), communicate with on-device software 114, generate and/or distribute software updates and automatic analysis protocols using, for example, medical-mathematical models, look-up tables, statistical learning models and/or neural network(s), monitor device performance and/or generate outputs.

The software system 112 may also include on-device software 114. The on-device software 114 is software provided on a monitoring device 100 (e.g. in the microprocessor 109) that the monitoring device can connect to via an interface 111. The on-device software may include software(s) to control the device 100, generate outputs, distribute the patient measurements and/or assess and/or analyse the patient measurements, including clinically useful measurements, taken by the sensor(s) 101, including through the use of one or more automated analysis protocols. Automated analysis protocols on the on-device software 114 may be derived from e.g. mathematical medical models, look-up tables, statistical learning models and/or neural network(s) to analyse the clinically useful data. Communication to and from the on-device software 114 and/or between various components of the system (e.g. server 113) may take place over a communications network such as the internet.

In particular, the on-device software 114 can be arranged to use the measurements to allow for calibration to, and the accommodation of, different skin property/properties (as measured by the device 100).

The measurement and analysis software 200 uses both AI (artificial intelligence) and non-AI models, which may include medical-mathematical models, look-up tables, statistical learning models and/or neural network(s), (which are trained and developed at the remote device 300) to analyse the patient data at the point of test (i.e. within the monitoring device 100) to deliver a clinically relevant output to the user 400 on the user interface 110.

The measurement and analysis software 200 can also encrypt and store the measured patient data on the monitoring device 100, as well as sending encrypted anonymous data measured by and from the monitoring device 100 to the remote device 300.

The measurement and analysis software 200 thus interfaces with both the monitoring device 100 (or its sensor(s)) and the remote device 300, as illustrated in Fig. 1.

User input to the device 100 (via the user interface 110) initiates a monitoring process to measure patient data from a patient with the device 100. This patient data is provided by the monitoring device 100 to its measurement and analysis software 200 for encryption, storing in the monitoring device 100, analysis and (if/when possible/desired/agreed to) transmission to a remote device 300.

The measurement and analysis software 200 can also receive from the remote device 300 updates to its software, protocols and/or models. An output (analysis and interpretation result) of the measurement and analysis software 200 is provided, via the user interface 110 on the device 100, to the user 400.

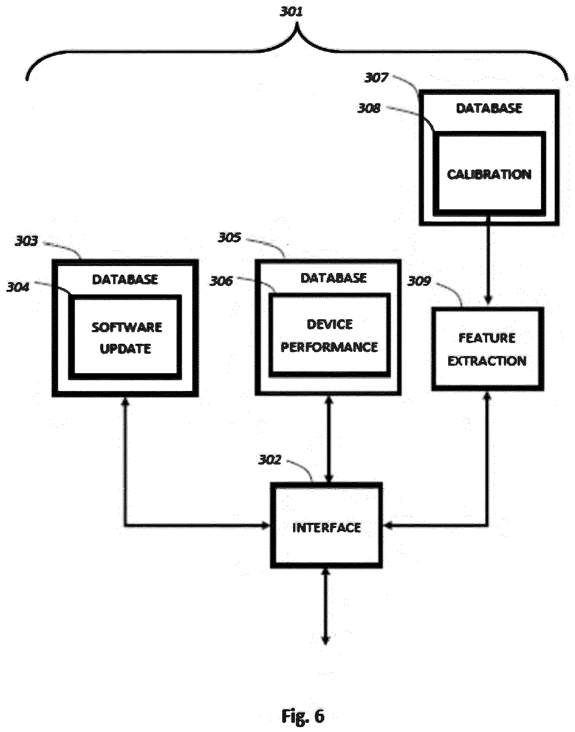

The remote device 300 contains a machine learning system 301 (see Fig. 6), which can provide updates to the models and/or analysis protocols, which may include medical-mathematical models, look-up tables, statistical learning models and/or neural network(s), used in the measurement and analysis software 200 on the monitoring device 100. The machine learning system 301 comprises machine learning software, non-machine learning software and a data repository 305 for storing data received from the monitoring device 100, as controlled/directed by the measurement and analysis software 200.

The intended users of the measurement and analysis software 200 are users who operate the monitoring device 100 at the point of test. The measurement and analysis software 200 provides an easily understandable interface through which an operator can:
- select analysis protocols to be applied, e.g. measurement only, or measurement and subsequent interpretation;
- initiate reading and application of analysis protocols;
- determine output format, e.g. a traffic-light form giving an indication of a patient's health status, or a number on a pre-defined scale;
- control data management, e.g. digital export of readings;
- provide additional data depending on the specified protocol, e.g. age, height and/or other input patient data;
- initiate updates of the local models and/or analysis protocols in the measurement and analysis software 200 from the machine learning system 301 on the remote device 300.

The measurement and analysis software 200 can provide or control the following functionalities:
- device control and measurement;
- measurement analysis and interpretation;
- data storage; and
- connection with the machine learning system 301 on the remote device 300.

The main functionality of the measurement and analysis software 200 can be to apply models and/or analysis protocols offline at the point of test and provide formatted readings of the measurements taken by the monitoring device 100 to the user as well as an interpretation report of those measurements, giving an indication of the patient's health status.

The measurement and analysis software 200 may receive measurements pre-processed by the one or more MCUs/processor(s) 106 directly from within the monitoring device 100 as well as user input data (e.g. by the user or patient) entered through the user interface110. The monitoring and analysis software 200 may further receive measurements that are not pre-processed directly from within the monitoring device 100.

Input patient data may include clinical information known about the patient, e.g. diagnosed status, and/or patient measurement histories from prior interactions with the system. According to specified models and/or analysis protocols, the measurement and analysis software 200 may apply analytical processing to the measurements received, e.g. adjust measurements for skin properties such as phototype/pigmentation. These measurements are then analysed offline by the measurement and analysis software 200 according to models and/or analysis protocols which are defined by and downloaded from the machine learning system 301 on the remote device 300. The analysis includes the application of trained models and/or analysis protocols to the measurements, which may be pre-processed, according to each of a number of analytic modes which may comprise an indication of one or more disease(s) and/or a spectral classification.

The measurement and analysis software 200 interprets the output of this analysis according to predefined models and/or interpretation protocols which are defined by and downloaded from the machine learning system 301 on the remote device 300 and presents the findings to the user on the user interface 110.

In one embodiment, the output of the analysis is presented to the user in a traffic-light form indicating the status of the patients' health, in response to which the operator and/or patient can take appropriate further action such as seeking further medical advice, or scheduling or arranging future monitoring.

The measurement and analysis software 200 also handles the encryption and storage of local measurements made by sensors in the monitoring device 100.

The patient's data is encrypted, anonymised and compressed for storage in the monitoring device 100.

There are one or more types of data which can be stored by the measurement and analysis software 200 on the monitoring device 100. In one embodiment, three types of data can be stored:
(i) patient measurements and input patient data;
(ii) user activity log, e.g. a file to where user login information and activity messages are written and stored;
(iii) system and model, analysis protocol and/or interpretation protocol update log and version information.

In some embodiments, further data may also be stored if appropriate or useful.

The measurement and analysis software 200 is or can be periodically (e.g. when connected) updated from the machine learning system 301 when improved models, analysis protocols and/or interpretation protocols are available.

The measurement and analysis software 200 is able to connect securely to the machine learning system 301 with an established network connection, which may be via a wired connection, Wi-Fi or a mobile data network, for example.

Through this connection, the measurement and analysis software 200 may be able to log into an automated access gateway on the remote device 300, where an integrity check of the connecting measurement and analysis software 200 is performed. Local encrypted data on the monitoring device 100 is uploaded to the remote device 300 through an established and verified connection. The connection is also used to download model, analysis protocol and/or interpretation protocol updates made available by the machine learning system 301 on the remote device 300.

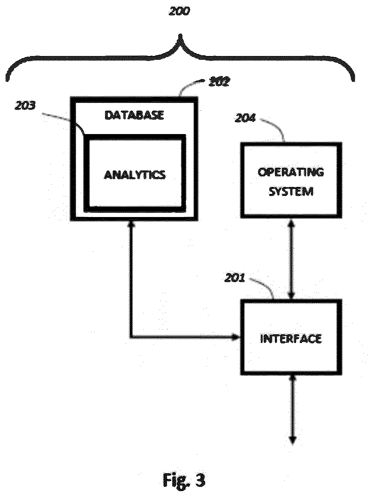

Measurement and analysis software 200 is illustrated in Fig. 3 and may include software(s) to control the device, generate outputs, distribute the patient measurements and/or assess and/or analyse the patient measurements, including through the use of one or more automated analysis protocols.

The interface 201 on the measurement and analysis software 200 can operate as a secure interface that allows for communication between (to and from) one or more components of the measurement and analysis software 200 and one or more other components in the system including the monitoring device 100 and/or machine learning system 301.

A database 202 is provided as part of the measurement and analysis software 200 that may contain patient data, data that may have been detected, measured, assessed and/or analysed by the monitoring device and/or the measurement and analysis software 200 and/or additional data. The database 202 may store the data temporarily or permanently. Analytics 203 comprises one or more automated analysis protocols, which may have been developed using medical-mathematical models, look-up tables, statistical learning models and/or neural networks, for analysing patient data and/or additional data in the database 202 and providing an output (e.g. patient measurement and/or analysis result). The analytics 203 can be updated periodically when the on-device software 200, through the on-device software interface 201 and software provider interface 302, is connected to the machine learning system 301.

An operating system 204 is provided as part of the measurement and analysis software 200 for controlling and operating the monitoring device, providing or performing device operator authentication, and/or analysing of the output (measurement) from the one or more sensor(s) on the monitoring device 100. The operating system 204 may, through the interface(s) of the measurement and analysis software 201 and the software provider 302, communicate data (e.g. device performance data 305) with (to and from) the machine learning system 301.

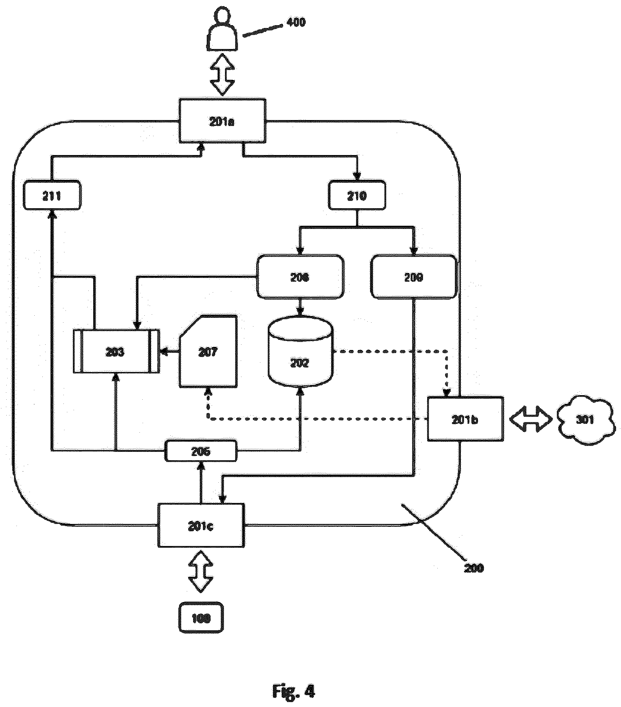

Fig. 4 is a flowchart of an embodiment of the measurement and analysis software 200 operational workflow. Solid lines show user interaction with the measurement and analysis software 200. Dashed lines show data flow between components of the measurement and analysis software 200 and the machine learning system 301 on the remote device 300.

The user 400 interacts with the measurement and analysis software 200 via a user interface 201a. In the case of the device 100, the user interface 201a may consist of a screen and/or control panel.

User input 210 that may be input via the user interface 201a can be split into analytic inputs 208 (which control the analysis of measured data) and control inputs 209 (which control the measurement process, i.e. obtaining measured data with the sensors of the device 100).

The control inputs 209 can be sent to a device interface 201c to control (e.g. initiate) the measurement process performed by the device 100.

After a measurement process has been performed by the device 100, measured data 206 obtained from the measurement process (i.e. measured by the one or more sensors of the device 100) may be output from the device interface 201c.

The measured data 206 is stored in local data storage 202. When the measurement and analysis software 200 is connected to the machine learning system 301 via, for example, a remote device interface 201 b, the measured data 206 can be sent to the machine learning system 301 for storage and/or analysis, e.g. to provide software, model and/or protocol updates 207.

The measured data can also be output at step 211 to the user interface 201a (where it may be, for example, displayed on the user interface 110 of the device 100).

In addition, the measured data 206 can be input to analysis software 203. The analytic inputs 208 can also be input to the analysis software 203 and the measured data is then analysed according to the analytic inputs 208 and analysis software model and/or protocol updates 207 (previously) received from the machine learning system 301 via a remote device interface 201b. The output of the analysis software 203 can be output at step 211 to the user interface 201a (where it may be, for example, displayed on the user interface 110 of the device 100).

The machine learning system 301, which is stored on the remote device 300, will now be described in more detail.

The machine learning system 301 is a multi-component patient data analytics and clinical decision support system. The machine learning system 301 is a stand-alone tool accessible through a secure gateway, as well as accessible directly by the measurement and analysis software 200 on the device 100.

The machine learning system 301 consists of one or more types of components and, in one embodiment consists of three types of components which can include:
- Access gateways: These can be the various ways that the machine learning system 301 can be accessed by users and/or software.
- Data repositories 307: These can receive anonymised measured data from the device 100 through the analysis software 200, and can be accessible directly by authorised users.
- Analysis components which, in one embodiment, may include one or more of the following:
   ∘ Database Analytics: the machine learning system 301 can analyse a central data repository 307 of measured data measured by a device (or devices) 100 on a number of patients (or a subset thereof) and produces statistical summaries and visualisations of selected data.
   ∘ Spectral Analysis: the machine learning system 301 can perform analysis of spectral data measured by and received from a device(s) 100 and produce models, analysis protocols and/or interpretation protocols using the spectral data for use as a tool in the measurement and analysis software 200 that may detect and/or assess life-threatening infectious and/or cardiometabolic factors and/or conditions.
   ∘ Pattern Analysis: the machine learning system 301 may use the patient data (measured data) in the central data repository 307 to find patterns which separate different patients according to health status, and develop and/or deploy models, analysis protocols and/or interpretation protocols based on those patterns to classify new patients.
   ∘ Interpretation: the machine learning system 301 allows for the development and deployment of measurement interpretation protocols.

A machine learning system (or software provider) 301 is illustrated in Fig. 6. It comprises one or more databases and/or software(s) which can store data, communicate with the measurement and analysis software 200, generate and/or distribute software updates 304 and automated analysis protocols developed using, for example, medical-mathematical models, look-up tables, statistical learning models and/or neural network(s), monitor device performance data and/or generate outputs.

The machine learning system 301 has an interface 302 which can be used to communicate and/or share data, outputs and/or automated analysis protocols securely between one or more components of the machine learning system 301 and one or more other components in the system (e.g. the measurement and analysis software 200). Access to the interface 302 may occur via a wired or wireless connection. One or more users may be able to access the machine learning system 301 through the interface 302 at the same time.

The machine learning system 301 has a database 303 that can store one or more software versions and/or software updates 304 which may include software updates for the measurement and analysis software 200 such as updates to the operating system 204.

The machine learning system 301 has a database 305 that can store and/or assess data on device performance 306 from one or more monitoring devices. Device performance 306 comprises data about the one or more monitoring devices in use. This may include device usage, device authentication, device performance and/or function of the one or more sensor(s), user interface and/or microprocessor of the monitoring device(s), and/or data on monitoring device(s) calibration for measurement reliability and repeatability.

A database 307 is provided in the machine learning system 301 that can store one or more types of data for calibration or adjustment 308 (e.g. patient data and/or other data). Calibration 308 may include patient data and/or other data that may be used by the feature extraction 309 to generate and/or update the automated analysis protocols (e.g. using medical-mathematical models, look-up tables, statistical learning models and/or neural network(s)) and/or to generate other outputs (e.g. a report).

Feature extraction 309 comprises extracting data characteristics from calibration 308 to generate automated analysis protocols and/or output automated analysis protocols. Automated analysis protocols may be generated using medical-mathematical models, look-up tables, statistical learning models and/or neural network(s).

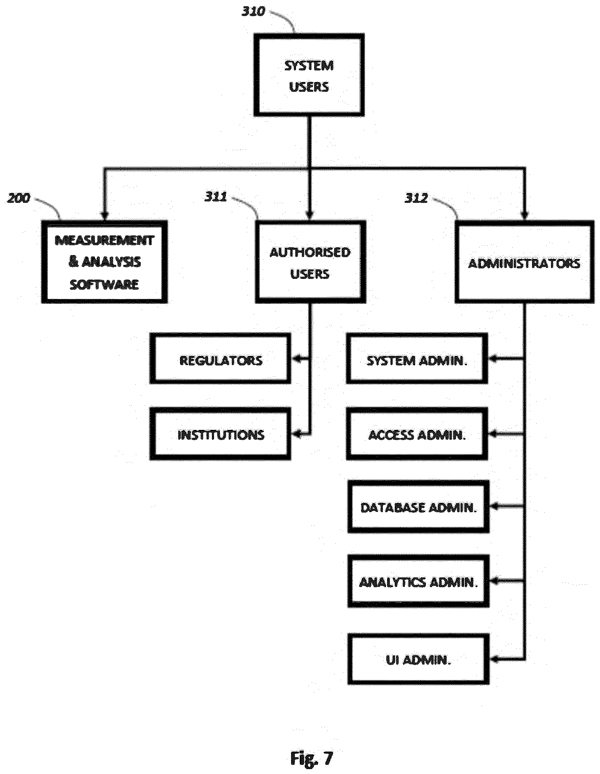

As illustrated in the schematic diagram in Fig. 7, the intended users 310 of the machine learning system 301 can be separated into one or more categories and, in some embodiments are separated into three categories: the measurement and analysis software 200, authorised users 311 and administrators 312. Each of these user categories may have different access gateways or access points to the machine learning system 301, of which one embodiment is described below, with different degrees of access rights.

The administrators 312 can be a class of users which have full access rights to core parts of the machine learning system 301 and may develop or change the functionality of the machine learning system 301.

The administrators 312 can be broken down into sub-categories with, for example, the following sub-categories:
∘ System Administrators: Supervising technical system integrity of the machine learning system 301 and its hosting server. System administrators may not have read or write privileges for the machine learning system 301 including its associated data repository 307 on the remote device 300. System administrators can be thought of as administrators provided by the host of the machine learning system 301 (the remote device 300) to ensure that the system can be successfully hosted on the particular server system.
∘ Access Administrators: Administrators of access authorisations. This type of administrator can appoint new administrators as well as grant authorisations to users.
∘ Database Administrators: A primary function of this type of administrator may be to design, deploy and/or maintain the databases within the machine learning system 301 with full read and write privileges exclusively to the data repository 307 of the system.
∘ Analytics Administrators: Analytics administrators may design, deploy and/or maintain database analytics, models, analysis protocols and/or interpretation protocols. They may have full access rights to all analysis components of the system as well as read privileges of the data repository 307.
∘ UI (user interface) Administrators: This type of administrator may design, deploy and/or maintain client access gateways, i.e. a web interface used by authorised users and an automated access interface used by the measurement and analysis software 200 (e.g. the remote device interface 201b). UI administrators may have read access only to the data repository 307 and the analytical components.

The authorised users 311 may be clients who can directly access the machine learning system 301 through a designated access gateway and use the various components, in accordance to their authorisation, of the system directly, without being able to develop or change its functionality. This is intended, for example, for institutional access exemplified by health systems, service-providers, policy makers, or donors.

The machine learning system 301 interacts directly only with the measurement and analysis software 200 through an automated access gateway. Through this gateway, the measurement and analysis software 200 can be the intermediary between the machine learning system 301 and the monitoring device 100.

The relationship between the machine learning system 301 and the measurement and analysis software 200 can be viewed as that of a trading relationship. The machine learning system 301 can provide models, analysis protocols and/or interpretation protocols for assessment and/or detection of a health status of a patient which the measurement and analysis software 200 receives and provides newly measured data in return.

As described above, one component (type) of the machine learning system 301 is access gateways. The term "access gateways" refers to one or more intended access points for all users and administrators to the machine learning system 301. As different users or administrators may require access to various levels of the system, the different gateways are intended to allow setting those limitations. These different access points can grant access appropriate to their system criticality. Whereas some access gateways provide superficial access through a web interface, others may grant access directly to the data repository 307 or analysis code. In addition to providing and limiting users' access to various levels of the system, the access gateways can be thought of as workspaces for the different user classes.

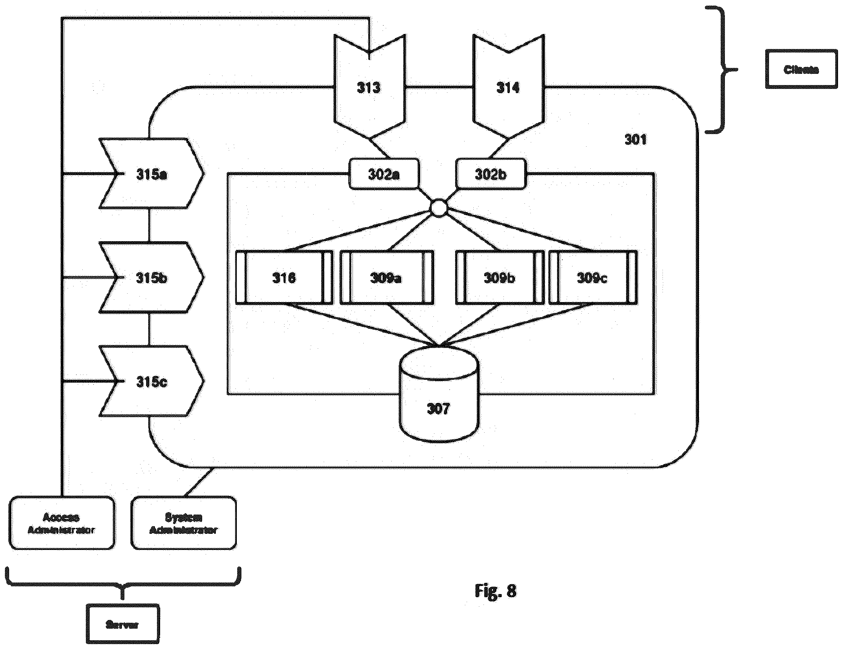

The access gateways are linked to different user types as illustrated in Fig. 8.

One access gateway to the machine learning system 301 is an access gateway for authorised users 313. This gateway 313 provides access to the machine learning system 301 for authorised users 311. The access gateway for authorised users 313 may link to one or more web interface(s) 302a.

A further access gateway can be an access gateway 314 for the measurement and analysis software 200.

The access gateways 313 and 314 can be viewed in one embodiment as client access gateways.

Further access gateways (e.g. 305a-c) can be provided for administrators which may include UI administrators, analytics administrators and database administrators, respectively. The access gateways 305a-c can be viewed as system access gateways.

The web interface 302a provides an online portal that may allow authorised users 311 access to the machine learning system 301 through a username and password protected web interface. The web interface 302a allows authorised users 311 to query the data repository 307 through the gateway for authorised users 313 for patient data and other information contained therein. The web interface 302a does not allow editing of the data stored in the data repository 307 in any way. However, the web interface 302a does allow authorised users 311 to download fully anonymised data from the data repository 307 via the gateway for authorised users 313.

Authorised users 311 can run pre-formed statistical summary scripts that may be defined by a database analytics component 316 as well as develop and run their own through the web interface 302a. The web interface 302a allows for data visualisation through pre-formed as well as user-defined scripts producing outputs (e.g. statistically useful plots).

Models and/or protocols produced by the components of the machine learning system 301 can be run from the web interface 302a. However, the models and/or protocols cannot be inspected for architecture or weights, nor can they be changed or downloaded.

The access gateway 314 may link to one or more automated access gateway 302b which can process pull and push requests by the measurement and analysis software 200 using a queueing system for simultaneous access requests. The one or more automated access gateway(s) 302b perform security checks for verifying the integrity of the connecting measurement and analysis software 200. Once a genuine connection has been established, the access gateway 314 can accept data to be transferred onto the machine learning system 301, and make updated and encrypted model and/or protocol weights available for download. Any received data will be pushed to a quarantine area of the data repository 307. No data can be downloaded from the data repository 375 using the access gateway 314.

The interface access gateway 315a can be for UI administrators and give access to the functionality level of the automated access gateway 302b and web interface 302a described above. This can allow UI administrators full control over interface development for clients (authorised users 311 and the measurement and analysis software 200), and may include write/remove privilege of the interface code, intended for monitoring, error resolution and updating. The interface gateway 315a also grants all data and model usage privileges granted to web interface 302a users.

The analytics access gateway 315b can allow analytics administrators to develop and integrate analytics code. This access gateway 315b can give full access to the core analysis component of the system which, in one embodiment may include four core analysis components of the system, those being, for example the database analytics component 316, a spectral analysis component 309a, a pattern analysis component 309b and an interpretation protocol component 309c.

The analytics access gateway 315b can allow analytics administrators to test and implement new analysis models, protocols and/or scripts as well as monitor, bug-fix and/or update existing ones. The analytics access gateway 315b also may grant all data and model usage privileges granted to web interface 302a users as well as unconstrained read privileges of the data repository 307.

The database administrators access gateway 315c may be a gateway on the server for database administrators. This gateway 315c may allow for full control over the database management system for all components of the data repository 307. This gateway 315c can allow database administrators to develop and/or maintain database schemas which feed into the machine learning system 301. In one embodiment, it may be that data can only be removed or edited through the access gateway 315c.

Both access administrators and system administrators may be granted access to use any of the above gateways according to specific needs.

In other embodiments, the breadth of access through each gateway may be slightly different to that described above.

In other embodiments, a separate access gateway for system administrators is provided, to monitor the technical integration of each component with one or more host server(s), rather than read/write privileges to analysis components 316, 309a, 309b, 309c.

In other embodiments, the access provided by the automated access gateway 314 for the measurement and analysis software 200 is altered according to specific encryption and security protocols that may be used.

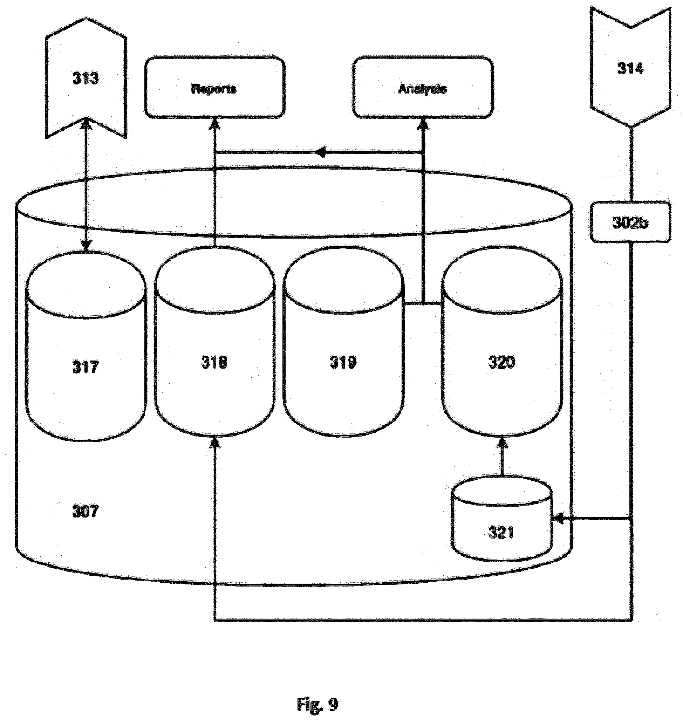

The data repository 307 is illustrated in more detail in Fig. 9.

The data repository 307 serves as a central data repository for the machine learning system 301 and can store anonymised data. The data repository 307 includes one or more databases 319 for data from one or more datasets and/or one or more data processing and analysis pipelines as well as one or more databases 320, 321 for data collected by the monitoring device(s) 100 and uploaded through measurement and analysis software 200.

The data repository 307 may also include safeguards to ensure that newly uploaded data is compatible with the existing databases.

The data stored in the data repository 307 can be used to develop and train (e.g. machine learning) models and/or protocols to assess and/or detect the health status of a patient provided by the measurement and analysis software 200 on the monitoring device(s) 100.

Authorised users 311 can have access to the data repository 307 via access gateway 313 and may be able to query the data repository 307 directly.

The data repository 307 stores anonymised analytics data in one or more databases 319, 320, 321, and user data and other metadata in database(s) 317 (e.g. patient data).

The data repository 307 includes databases 319 for data from one or more datasets and/or one or more data processing and analysis pipelines (e.g. collected during clinical trials) as well as databases 320, 321 for data collected by the monitoring device(s) 100 and uploaded through the measurement and analysis software 200. The databases 320, 321 may include (or have associated) safeguards to ensure that newly uploaded data is compatible with the existing databases. The data stored in the databases 319, 320, 321 can be used to develop and train (e.g. machine learning) models and/or protocols to assess and/or detect the health status of a patient performed by the measurement and analysis software 200.

Additional one or more databases 317 and 318 in the data repository 307 can store encrypted user login and password information as well as metadata on system activity and interactions with the measurement and analysis software 200.

The databases 317, 318, 319, 320, 321 in the data repository 307 may be organised through a relational database management system (RDBMS).

The database 319 can store data obtained from one or more datasets and/or one or more data processing and analysis pipelines. Data stored in this database 319, which may be of high quality and/or high fidelity may form a core for any model and/or protocol derived from the data repository 307. This one or more database(s) 319 may be maintained by database administrators, and may be readable by analytics administrators.

A quarantine database 321 can store newly uploaded data from the measurement and analysis software 200. This quarantine database 321 may serve as a safety barrier for uploaded data before it is merged into the one or more database(s) 320, to ensure that uploaded data is compatible with and will not corrupt other parts of the data repository 307. Either automatic or manual review by a database administrator may be necessary to confirm the data and merge it into the one or more database(s) 320. Data reviewed in this way can be marked as unsafe or incompatible (if applicable) and be deleted. The one or more database(s) 320 can be accessible to and maintained by database administrators only.

The one or more database(s) 320 stores reviewed and confirmed data which was uploaded by the measurement and analysis software 200 and successfully passed through the quarantine database 321. This one or more database(s) 320 is the final storage location for data recorded in the field by monitoring device(s) 100. The data of the one or more database(s) 320 is optionally used in model and/or protocol generation (e.g. using medical-mathematical models, look-up tables, statistical learning models and/or neural network(s)) as specified by analytics administrators. The one or more database(s) 320 can be maintained by database administrators, and readable by analytics administrators.

The one or more database(s) 317 can store encrypted passwords and login information for authorised users. Anytime a new user is given access authorisation, their details can be stored here. Whenever a user logs into the system, their credentials can be checked against these one or more database(s) 317.

The metadata database 318 can store any other data captured by the machine learning system 301, such as user activity logs, measurement and analysis software 200 version history, system version control.

Data from the metadata database 318, and from the one or more databases 319 and 320 can be used for report generation.

The data repository 307 may include additional databases, for example to store patient and/or spectral data recorded by the monitoring device(s) 100 in separate databases.

As described above, the machine learning system 301 has a number of analysis components 316, 309a, 309b, 309c. One embodiment of these number of analysis components 316, 309a, 309b, 309c will now be described in more detail.

The database analytics component 316 can provide database analytics. It contains software that may specify a series of predefined methods to obtain summary statistics from the data repository 307 as well as from the models and/or protocols. These methods aim to generate visual and/or numerical summaries of the data repository 307.

The database analytics component 316 may include all non-machine learning analysis tools provided by the machine learning system 301.

Statistical summaries such as, but not exclusively, mean values, standard deviations, maximal ranges and modes of individual and combinations of patient data as well as other population measures (e.g. age, height, weight) may be produced by the database analytics component 316.

The database analytics component 316 can also produces visualisations (e.g. histograms, scatter plots, distribution functions) of selected patient data.

The spectral analysis component 309a uses spectral data, i.e. data obtained from spectroscopy measurements (e.g. made by the monitoring device(s) 100 or from one or more datasets and/or one or more data processing and analysis pipelines). This can be spectra (spectral data) obtained from vibrational spectroscopy measurements. This spectral data can be used to produce models and/or protocols which can then be used by the measurement and analysis software 200 to measure, assess and/or detect the unique spectral features representative of one or more disease(s) including life-threatening infectious diseases and/or cardiometabolic factors and/or conditions. The spectral analysis models and/or protocols are analysis and/or interpretation models and/or protocols which may be used to identify a category to which a newly measured spectrum may belong.

In one embodiment, the spectral analysis performed by the spectral analysis component 309a may be based on a one-dimensional convolutional neural network combining a series of convolutional and down-sampling layers to detect features in the spectral data before being connected to a series of fully connected layers which use the detected features for analysis and/or interpretation.

The forms of the convolutional filters, i.e. the network weights, can be obtained during training of the neural network on data from the one or more database(s) 319 using a number of feed-forward and back-propagation loops as a means to optimise neural network weights via gradient descent.

The architectural design of the neural network can be optimised by analytics administrators using for example neural network validation techniques such as k-fold cross-validation to establish the learning capacity of model and/or protocol designs on the available data whenever new model and/or protocol updates are developed.

After the architecture of the, for example, neural network is determined, the one or more models and/or protocols can be trained on all available data in the one or more database(s) 319 and its weights can be made available for download through the measurement and analysis software 200 and usable by authorised users 311.

The input to the models and/or protocols available for use are newly measured spectra. The output may contain information and/or data which may comprise a list of numbers between 0 and 1, where each number represents a health state and/or category and the value of each number may represent the probability of the input spectrum (spectral data) belonging to that category.

Each potential type of spectrum may be associated with its own convolutional neural network model. For example, a model for vibrational spectra may exist alongside a model for near-infrared spectra.

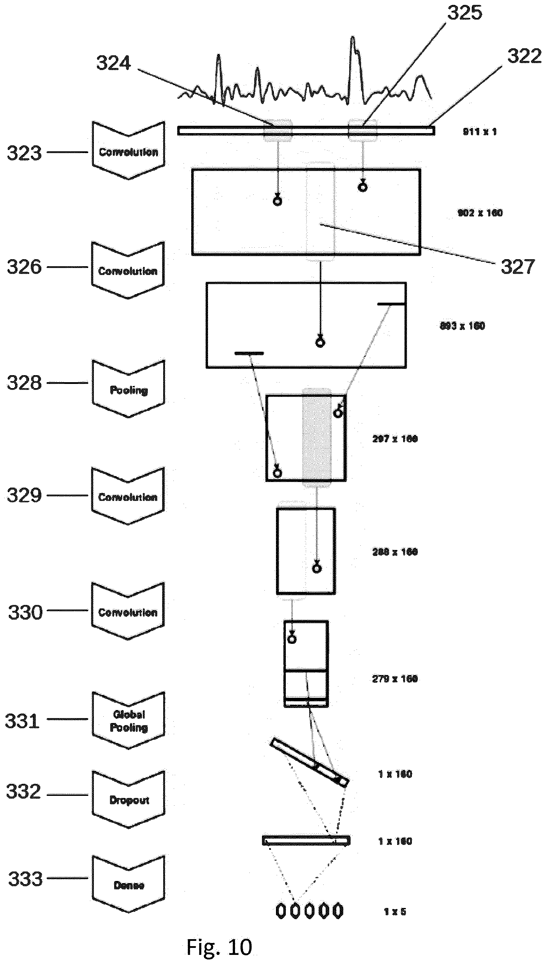

The architecture of an example convolutional neural network, which could be used in the spectral analysis component 309a, is illustrated in Fig. 10.

In this example, the input 322 consists of a clean spectrum measured in 911 frequency bins, and is thus a vector of dimensions 911 x 1.

A convolutional layer 323 can then take this input and applies 160 different convolutional kernels (or filters) of length 10 to the input. As a filter is slid across the input vector, it can produce numbers stored as a column in the output matrix, such that each different filter represents a different column in the output matrix, as illustrated by the shaded filters 324, 325 mapping to different columns in the input to the next convolutional layer.

Another convolutional layer 326 can use the output from the first layer 323 as an input and apply its own set of 160 filters to the results of the first convolution 211. In a one-dimensional convolutional neural network, the filter width is set to the width of the input, and here their length is set to 10, as shown as the shaded filter 327.

The next layer 328 is a max-pooling layer, which retains only the maximal value of each previous filter (i.e. column of the input) within its own filter height. This filter can be applied in a non-overlapping fashion across the input in order to divide the input size by a factor of three in this example.

Two more convolutional layers 329, 330, equivalent to those applied in the first two layers 323, 326 may be applied for more complex feature detection.

In this example, the output of the fourth (overall) convolutional layer 330 is then flattened by applying global average pooling 331 where the average value of each of the previous filter outputs is retained.

This can create a vector of dimensions 1 x 160, which can be used in a series of dropout layers 332, to prevent over fitting, and dense layers 333 to arrive at a vector of probability measures to classify the input spectrum into, in this example, one of five categories.

The architecture depicted in Fig. 10 and described above is just an example. The precise architecture of the network can be chosen so as to optimise analysis and/or interpretation capabilities of the measurement and analysis software 200.

The extent of the classification categories can depend on (and be defined by) the number of differently classified training spectra obtained from one or more datasets and/or one or more data processing and analysis pipelines.

The pattern analysis component 309b contains a pattern classification component to find patterns related to the health status of a patient.

The pattern analysis component 309b can analyse a range of data (e.g. patient data) measured by the monitoring device 100 and/or input patient data for a particular patient and may assign a classification and/or category to the data. The pattern analysis component 309b will use the combination of data from the one or more database(s) 319 320 to find patterns in the data which it associates and/or labels with categories related to the health status of a patient and ultimately produces models and/or protocols able to associate new patient data and/or input patient data with a particular category.

A protocol for categorising the one or more databases 319, 320 containing patient data can be defined. This protocol may be applied to the data repository 307 by analytics administrators to assign categories and/or labels to the data.

Once the data is labelled, one or more models and/or protocols can be trained on all data points. These one or more models and/or protocols can take data (e.g. patient data, input patient data) (for a particular patient), measured by the monitoring device 100, as input and produce as an output information and or data which may comprise a list of numbers between 0 and 1, where each number represents a category that relates to the health status of a patient and the value of each number may represent the probability of the data (e.g. patient data, input patient data) belonging to that category.

The interpretation protocol component 309c can define models and/or protocols for how information processed by the various other analysis components can be interpreted and delivered to the user of the measurement and analysis software 200.

The machine learning system 301 acts as the platform on which these models and/or protocols are developed and sent out to the measurement and analysis software 200 as one or more software updates.

Interpretation protocols use patient data from the monitoring device 100, which may be pre-processed, and outputs from pattern analysis and/or categorisation, and/or spectral analyses as inputs. Interpretation models and/or protocols can provide an indication of the patient's health status.

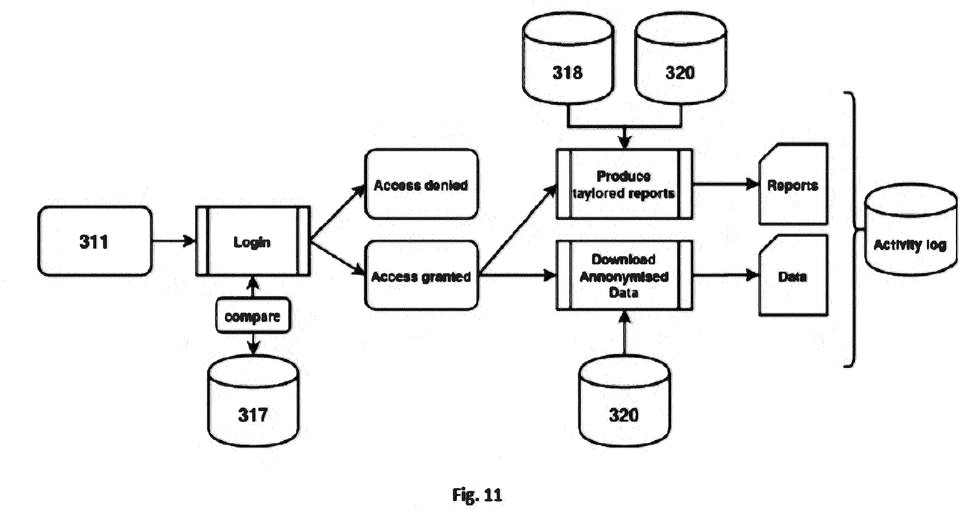

Fig. 11 is a schematic flowchart illustrating one example of use of the machine learning system 301 by an authorised user 311.

In the example in Fig. 11, first, the authorised user 311 has to login to the machine learning system 301. The authorised user 311 enters their login credentials (e.g. username and password) to the remote device 300 on which the machine learning system 301 is hosted. The entered details are compared with authorised user details stored in the database 317. If a match is found, then access is granted and the authorised user 311 is logged in to the machine learning system 301. If a match is not found, then access is denied.

If access is granted and the authorised user 311 is logged in, the authorised user 311 can produce outputs (e.g. tailored reports) and/or download anonymised data. The outputs can be produced from metadata from the one or more database(s) 318, 320. The anonymised data comes from the database 320. An activity log can be created recording the use of the machine learning system 301 by the authorised user 311.

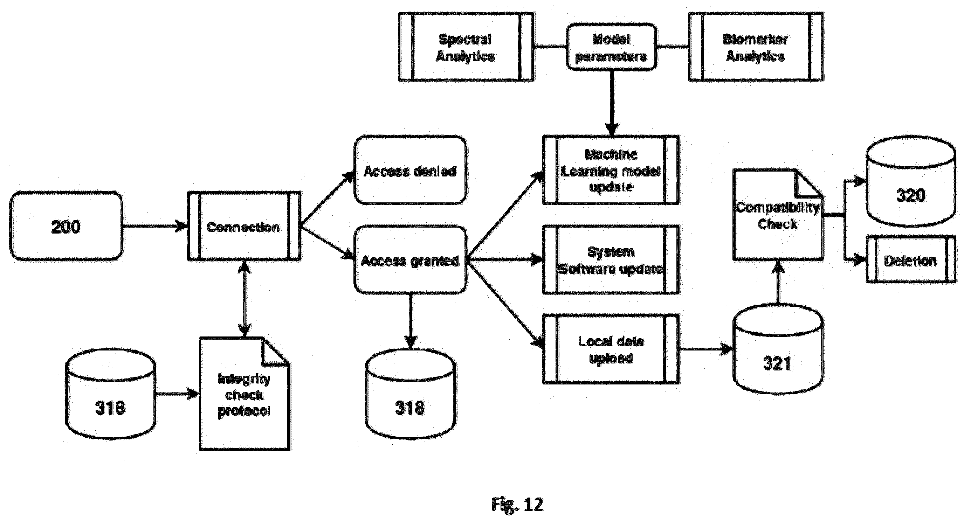

Fig. 12 is a schematic flowchart illustrating one example of automated measurement and analysis software 200 use of the machine learning system 301.

In this case, when measurement and analysis software 200 seeks or requests connection to the machine learning system 301, an integrity check protocol is followed using measurement and analysis software metadata stored in the database 318. Following this integrity check, access to the machine learning system 301 is either granted to the measurement and analysis software 200 or denied. If access is granted, this is recorded in a system activity log stored in the database 318.

If access is granted, the measurement and analysis software 200 can, for example:
∘ request a model update (e.g. machine learning) from the machine learning system 301;
∘ request an analysis and/or interpretation protocol update from the machine learning system 301; and/or
∘ upload measured data from the monitoring device 100 on which the measurement and analysis software 200 is stored to the machine learning system 301.

In most cases, all three of these steps will be performed.

If a model update and/or an analysis and interpretation protocol update is requested, before the latest model and/or analysis and interpretation protocol is/are sent to the measurement and analysis software 200 on the monitoring device 100, it is first checked whether an update is needed/possible. If the versions of the model and/or an analysis and interpretation protocol on the monitoring device 100 correspond to those available from the machine learning system 301, then no update is sent as none is available. On the other hand, if the versions differ then an update can be sent to the measurement and analysis software 200 on the monitoring device 100.

The model update consists of providing updated model parameters, which are provided by the spectral analysis component 309a and pattern analysis component 309b of the machine learning system 301.

If the measurement and analysis software 200 uploads patient data from the monitoring device 100 on which the measurement and analysis software 200 is stored to the machine learning system 301, this uploaded data can first be stored in the quarantine database 321. Next, a compatibility check can be performed to check that the uploaded data is compatible with the data already stored in the one or more database(s) 320. If the uploaded data is found to be compatible, then it is moved from the quarantine database 321 to the one or more database(s) 320. If the uploaded data is found not to be compatible, then it may be simply deleted from the quarantine database 321.

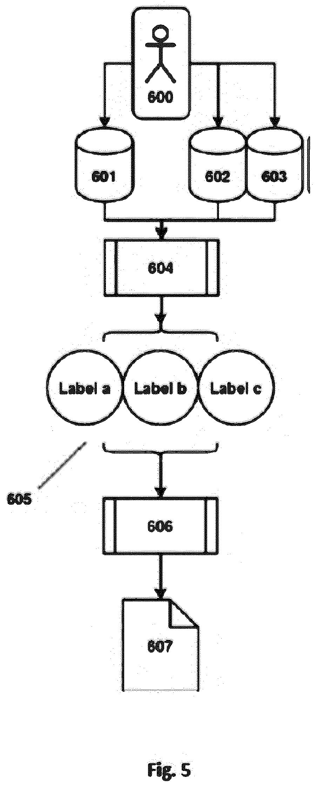

Fig. 5 illustrates one embodiment of analysis flow when performing measurement analysis with the analysis software 203 in the measurement and analysis software 200. The embodiment illustrated may correspond to the two types of analysis protocols described above, i.e. pattern analysis and spectral analysis, and may be conducted separately (e.g. one of pattern analysis or spectral analysis) or in combination (e.g. pattern analysis and spectral analysis) .

In Fig. 5 measured patient data 601, input patient data 602 and/or spectral data 603 can be obtained from patient 600 with/via the monitoring device 100. Next, the analysis software 604 can perform pattern analysis and/or spectral analysis and/or classification on the data 601, 602, 603 where each of data 601, 602, 603 can be used separately or in combination for the pattern analysis and/or spectral analysis, using pattern analysis models and/or protocols provided from the pattern analysis component 309b and/or spectral analysis models and/or protocols from the spectral analysis component 309a of the machine learning system 301, as described above. The result of the pattern analysis and/or spectral analysis done by the analysis software 604 may be information and/or data 605 (e.g. a series of numbers each being a percentage or number from 0 to 1) and representing the estimated or predicted health status of a patient and/or can be used to assess and/or detect the unique spectral features representative of disease(s), including life-threatening infectious diseases and/or cardiometabolic factors and/or conditions.

An interpretation protocol 606 can then applied to the information and/or data 605 to interpret the information and/or data 605 and provide an output report 607 to a user 600 of the monitoring device 100 and measurement and analysis software 200 (e.g. on the user interface 110 of the monitoring device 100). The interpretation protocol 606 can be provided from the interpretation protocol component 314 of the machine learning system 301, as described above.

Communication between (to and from) the components of the system (e.g. measurement and analysis software 200, software provider 301) may take place over a communications network such as the internet. Communication between components within a component of the system (e.g. the measurement and analysis software 200, software provider 301) may be wired and/or wireless.

## Claims

1. A monitoring device for measuring patient data from a patient, the monitoring device comprising measurement analysis software for analysing the patient data, and the monitoring device being configured to:
a. send patient data to a software provider; and
b. receive one or more updates to the measurement analysis software from the software provider.

2. A monitoring device as claimed in claim 1, wherein the measurement analysis software is configured to display a result of analysing the patient data with the measurement analysis software on the monitoring device.

3. A monitoring device as claimed in any preceding claim, wherein the measurement analysis software is configured to provide an interpretation of a result of analysing the patient data with the measurement analysis software.

4. A monitoring device as claimed in claim 3, wherein the measurement analysis software is configured to display the interpretation on the monitoring device.

5. A monitoring device as claimed in claim 3 or 4, wherein the interpretation comprises an indication of a health status of the patient.

6. A monitoring device as claimed in claim 5, wherein the indication of a health status of the patient comprises an indication of disease severity for at least one disease and/or an indication of disease progression for at least one disease.

7. A monitoring device as claimed in any of claims 3 to 6, wherein the interpretation comprises an indication of a spectral classification.

8. A monitoring device as claimed in any preceding claim, further comprising software for encrypting and/or anonymising patient data.

9. A monitoring device as claimed in any preceding claim, wherein the patient data comprises spectral data and the measurement analysis software is configured to analyse the spectral data to obtain a skin phototype or pigmentation indication.

10. A software provider for providing measurement analysis software for a monitoring device for measuring patient data from a patient, the measurement analysis software being software for analysing the patient data, and the software provider being configured to:
a. receive patient data from the monitoring device; and
b. send one or more updates to the measurement analysis software to the monitoring device, wherein the one or more updates to the measurement analysis software are based on an analysis of the received patient data.

11. A software provider as claimed in claim 10, the software provider comprising a quarantine database for storing received patient data from the monitoring device.

12. A software provider as claimed in claim 10 or 11, wherein the software provider comprises software for analysing the patient data received from the monitoring device.

13. A software provider as claimed in claim 12, wherein the software for analysing the patient data received from the monitoring device comprises one or more machine learning algorithms.

14. A software provider as claimed in claim 12 or 13, wherein the software provider comprises a database of data from one or more datasets and/or one or more data processing and analysis pipelines, and the software for analysing the patient data received from the monitoring device is also configured to analyse the data from one or more datasets and/or one or more data processing and analysis pipelines and to provide the one or more updates to the measurement analysis software based on the analysis of the patient data received from the monitoring device and the analysis of the data from one or more datasets and/or one or more data processing and analysis pipelines.

15. A software provider as claimed in any of claims 10 to 14, wherein the one or more updates to the measurement analysis software comprise one or more updates to model parameters used by the measurement analysis software and/or one or more updates to protocols used by the measurement analysis software.

16. A health monitoring system, the system comprising:
a. one or more monitoring devices for measuring patient data from a patient, each monitoring device comprising measurement analysis software for analysing the patient data measured by the monitoring device; and
b. a software provider;
wherein the health monitoring system is configured such that the one or more monitoring devices can send patient data measured by the one or more monitoring devices to the software provider, and the software provider can provide one or more updates to the measurement analysis software to the one or more monitoring devices.

17. A health monitoring system as claimed in claim 16, wherein:
a. the one or more monitoring devices is/are the monitoring device of any of claims 1 to 9; and/or
b. the software provider is the software provider of any of claims 10 to 15.

18. A method of monitoring patient health, the method comprising:
a. measuring patient data from a patient with a monitoring device;
b. analysing the patient data with measurement analysis software;
c. sending patient data to a software provider; and
d. receiving one or more updates to the measurement analysis software from the software provider.

19. A method as claimed in claim 18, further comprising displaying a result of analysing the patient data with the measurement analysis software on the monitoring device.

20. A method as claimed in claim 19, further comprising providing an interpretation of a result of analysing the patient data with the measurement analysis software.

21. A method as claimed in claim 20, wherein the interpretation comprises an indication of a health status of the patient.

22. A method as claimed in claim 21, wherein the indication of a health status of the patient comprises an indication of one or more disease(s).

23. A method as claimed in any of claims 20 to 22, wherein the interpretation comprises an indication of a spectral classification.

24. A method as claimed in any of claims 18 to 23, further comprising encrypting and/or anonymising patient data.

25. A method as claimed in any of claims 18 to 24, wherein the patient comprises spectral data and the method comprises analysing the spectral data to obtain skin property(ies) data.

26. A method of providing a software update to measurement analysis software for analysing patient data measured by a monitoring device, the method comprising:
a. receiving patient data from the monitoring device; and
b. sending one or more updates to the measurement analysis software to the monitoring device, wherein the one or more updates to the measurement analysis software are based on an analysis of the received patient data.

27. A method as claimed in claim 26, the method further comprising quarantining the received patient data.

28. A method as claimed in claim 26 or 27, wherein the analysis of the received patient data comprises using one or more algorithms, models and/or protocols.

29. A method as claimed in any of claims 26 to 28, wherein the one or more updates to the measurement analysis software are also based on an analysis of data from one or more datasets and/or one or more data processing and analysis pipelines.

30. A method as claimed in any of claims 26 to 29, wherein the one or more updates to the measurement analysis software comprise one or more updates to model parameters used by the measurement analysis software and/or one or more updates to protocols used by the measurement analysis software.

31. A computer program product comprising computer readable instructions that, when run on a computer, is configured to cause one or more processors to perform the method of any of claims 18 to 30.
